# EUROPEAN PATENT APPLICATION

(11) **EP 4 755 905 A1**
(43) Date of publication of application: **10.06.2026**
(21) Application number: 24217825.9
(22) Date of filing: 05.12.2024
(51) Int. Cl.: C07K 14/47, A61K 38/00, C07K 16/00, C07K 16/24

(54) **TRUNCATED PROGRANULIN PROTEIN AND USES THEREOF**

(71) Applicant: Fraunhofer-Gesellschaft zur Förderung der angewandten Forschung e.V., 80686 München (DE)
(72) Inventor: Safarian, Schara, 60596 Frankfurt am Main (DE); Köllner, Sarah, 60596 Frankfurt am Main (DE); Kannt, Aimo, 60596 Frankfurt am Main (DE)
(74) Representative: Kuttenkeuler, David

(57) **Abstract**

The present invention relates to a truncated progranulin protein or a variant thereof and a fusion protein comprising the truncated progranulin protein or a variant thereof. Provided are further nucleic acids and vectors for the expression of the truncated progranulin protein and the fusion protein of the invention, recombinant cells and pharmaceutical composition comprising the truncated progranulin protein, as well as its use in lysosome-dependent protein degradation of a target protein.

## Description

### FIELD OF THE INVENTION

The present invention relates to a truncated progranulin protein or a variant thereof and a fusion protein comprising the truncated progranulin protein or a variant thereof. Provided are further nucleic acids and vectors for the expression of the truncated progranulin protein and the fusion protein of the invention, recombinant cells and pharmaceutical composition comprising the truncated progranulin protein, as well as its use in lysosome-dependent protein degradation of a target protein.

### DESCRIPTION

Next to diseases caused by infections through viruses or microorganisms, there are diseases caused by a combination of genetic, physiological, environmental and behavioural factors. Such diseases are cancer, diabetes, certain cardiovascular conditions or neurodegenerative diseases. Thousands of autologous proteins play a major role in the development and the clinical manifestation of these diseases and are therefore potential therapeutic targets for drug discovery. 85% of the human proteome are considered "undruggable", or intractable to traditional drug discovery efforts leading to a major challenge in developing therapeutics against these targets. These proteins lack easily identifiable binding pockets or catalytic active sites that can be functionally and selectively targeted with a small-molecule, or other therapeutic modality (1).

These undruggable proteins are for example protein complexes that are involved in scaffolding non-enzymatic functions and may not possess deep binding pockets that can be targeted to disrupt or stabilize protein interactions. Furthermore, also the identification of binding pockets that can be pharmacologically targeted with small molecules within intrinsically disordered proteins remains challenging (2).

Targeted protein degradation (TPD) is currently the most promising approach for degrading these undruggable proteins. One example for TPD is the use of small compounds like PROteolysis TArgeting Chimeras (PROTACs) and molecular glues to recruit diseasecausing proteins for rapid destruction mainly via the endogenous ubiquitin-proteasome system (UPS). PROTACs regulate protein function by degrading target proteins instead of inhibiting them, providing more sensitivity to drug-resistant targets and a greater chance to affect the non-enzymatic functions. Therefore, PROTACs show better selectivity compared to classic inhibitors (3).

PROTACs ally with the ubiquitin-proteasome system (UPS) to achieve a regulation of protein levels in cells and represent therefore a chemical knockdown strategy with rapidity and reversibility. This strategy results in a new and different biology compared to other gene editing tools by avoiding misinterpretations that arise from potential genetic compensation and/or spontaneous mutations (4).

Despite all known progress in screening compounds for TPD, there is still a need to find new degrader compounds with the ability to modulate protein target levels by hijacking protein degradation systems to induce degradation of the target.

Progranulin (PGRN) is an evolutionarily conserved, secreted glycoprotein that can be proteolytically processed into several ~6 kDa granulin peptides (granulins A, B, C, D, E, F, G). It is a growth factor involved in wound healing, inflammation, tumor growth, and neurodegeneration, although the basis for these effects is not fully defined. Effects of PGRN on neurite outgrowth or cell survival have been reported in different assays. However, in some studies, PGRN had no detectable effects on the survival of neurite outgrowth from cerebral cortical or hippocampal neurons. The most common inherited form of Fronto-Temporal Lobar Degeneration (FTLD) known stems from haploinsufficiency of progranulin (PGRN) due to mutations in the granulin (*GRN*) gene and exhibits TAR DNA-binding protein 43 (TDP-43) plus ubiquitin aggregates (5). While human haploinsufficiency results in FTLD-TDP, mice homozygous for a *GRN* null mutation exhibit little of no FTLD phenotype (5). Frontotemporal lobar degeneration (FTLD) is the most prevalent form of early-onset dementia after Alzheimer's disease (AD), and accounts for 20-25% of presenile dementias (6).

As yet, there is no consensus regarding PGRN action in the nervous system or the mechanism, whereby 50% reduction might lead to FTLD-TDP. A fraction of PGRN can be proteolytically processed to smaller GRN peptides, but the relative role of these fragments in FTLD-TDP is not known. PGRN binds to cortical neurons via its C-terminus and binds sortilin *(Sort1).* Sort1 is a type I trans-membrane sorting receptor belonging to the VPS10P-domain receptor family involved in binding, internalization, and cellular trafficking of ligands (8). In the central nervous system (CNS), sortilin is expressed by neurons and PGRN is most strongly expressed by activated microglial cells after injury. Sortilin rapidly endocytoses and delivers PGRN to lysosomes (5).

PGRN facilitates neuronal uptake and lysosomal delivery of prosaposin (PSAP), the precursor of saposin peptides that are essential for lysosomal glycosphingolipid degradation. PSAP or saposin deficiency is known to cause several distinct lysosomal storage disorders, including Gaucher disease, Krabbe disease and metachromatic leukodystrophy. Several receptors have been shown to mediate PSAP lysosomal trafficking, including the cationindependent mannose 6-phosphate receptor (M6PR), sortilin and LRP1. PGRN bridges the interaction between PSAP and sortilin and facilitates lysosomal targeting of PSAP via sortilin (6).

The VPS10 domain of sortilin consists of two structural modules, a ten-bladed beta-propeller structure at the N-terminus (residues 81-611) and a cysteine rich 10CC module (10CCa and 10CCb, residues 612-757) at the C-terminus. PGRN binds to the beta propeller region of sortilin through its C-terminal tail (7).

Logan *et al* disclose a protein transport vehicle (PTV):PGRN comprising a recombinant protein linking PGRN to a modified Fc domain that binds human transferrin receptor for enhanced CNS biodistribution. PTV:PGRN rescued various GRN-/- phenotypes in primary murine macrophages and human iPSC-derived microglia, including oxidative stress, lysosomal dysfunction and endomembrane damage (9).

WO2021133907A1 discloses progranulin variants and fusion proteins that comprise a progranulin variant and an Fc polypeptide. Methods of using such proteins to treat progranulin-associated disorders (e.g., a neurodegenerative disease, such as frontotemporal dementia (FTD)) are also provided herein.

US20230406898A1 discloses fusion proteins that comprise progranulin and an Fc polypeptide. Additional, methods are disclosed to use such proteins to treat progranulin-associated disorders (e.g., a neurodegenerative disease, such as frontotemporal dementia (FTD)).

US10357542B2 discloses compositions and methods for diagnosis and treatment of lysosomal storage diseases and their diagnosis and treatment, including Gaucher's Disease and Tay-Sachs disease, and particularly which utilize progranulin (PGRN), or active PGRN peptides, including atsttrin. The invention also provides animal models of lysosomal storage diseases, including Gaucher's Disease and Tay-Sachs disease, based on or including PGRN mutations including PGRN null mutants and PGRN gene knock outs.

US20220265770A1 relates to methods and compositions for treating a neurodegenerative disease. More particularly, methods are disclosed of treatment of neurodegenerative diseases using progranulin and progranulin polypeptides, and methods of treatment of neurodegenerative diseases using effectors, or combinations of effectors, that modify progranulin expression.

US20120039865A1 relates to methods for identifying compounds that modulate the interaction between progranulin and sortilin. Furthermore, methods are disclosed for modulating the interaction between progranulin and sortilin and the activity of progranulin and/or sortilin. Additional, methods are disclosed of treating or preventing a disease, disorder, or condition by modulating the interaction between progranulin and sortilin, the activity of progranulin and/or sortilin.

Despite all above progress, so far the prior art discloses an interaction between progranulin and sortilin and compounds that modulate this interaction for the treatment or prevention of progranulin-associated diseases, disorders or conditions such as neurodegenerative diseases.

It is an object of the present invention to provide new applications for progranulin proteins.

### BRIEF DESCRIPTION OF THE INVENTION

Generally, and by way of brief description, the main aspects of the present invention can be described as follows:
In **a first aspect,** the invention pertains to a truncated progranulin protein or a variant thereof, wherein said truncated progranulin protein is N-terminally truncated compared to amino acid sequence according to SEQ ID NO. 1.
In **a second aspect,** the invention pertains to a fusion protein comprising a truncated progranulin protein (or a progranulin fragment) fused to a non-progranulin protein, wherein the truncated progranulin protein or progranulin fragment is a protein according to the invention.
In **a third aspect,** the invention pertains to a fusion protein comprising:
   - an antigen binding region,
   - optionally linked by a linker region with
   - a progranulin domain comprising the sequence of the truncated progranulin protein or a variant thereof according to the invention,
   wherein the progranulin domain specifically binds to sortilin.
In **a fourth aspect,** the invention pertains to a nucleic acid sequence encoding the truncated progranulin protein according to the invention or the fusion protein according to the invention.
In **a fifth aspect,** the invention pertains to a vector comprising the nucleic acid sequence according to the invention, preferably an expression vector, more preferably comprising a promoter sequence operably linked to the nucleic acid sequence according to the invention.
In **a sixth aspect,** the invention pertains to a host cell comprising the truncated progranulin protein according to the invention, the fusion protein according to the invention, the nucleic acid sequence according to the invention, or the vector according to the invention, preferably a host cell having the nucleic acid sequence according to the invention integrated in its genome.
In **a seventh aspect,** the invention pertains to a pharmaceutical composition comprising:
   (i) the truncated progranulin protein according to the invention, the fusion protein according to the invention, the nucleic acid sequence according to the invention, the vector according to the invention, or the host cell according to the invention, and
   (ii) a pharmaceutically acceptable carrier, stabilizer and/or excipient.
In **an eighth aspect,** the invention pertains to a compound for use in the treatment of a disease, the compound being selected from the truncated progranulin protein according to the invention, the fusion protein according to the invention, the nucleic acid sequence according to the invention, the vector according to the invention, the host cell according to the invention, or the pharmaceutical composition according to the invention.
In **a nineth aspect,** the invention pertains to a kit comprising the truncated progranulin protein according to the invention, the fusion protein according to the invention, the nucleic acid sequence according to the invention, the vector according to the invention, the host cell according to the invention, or the pharmaceutical composition according to the invention.
In **a tenth aspect,** the invention pertains to a method for producing the truncated progranulin protein according to the invention or the fusion protein according to the invention, comprising the steps of:
   (a) culturing and harvesting a host cell according to the invention under conditions suitable for protein expression; or
   (b) adding the nucleic acid sequence according to the invention, or the vector according to the invention to a cell-free protein synthesis system under conditions suitable for protein expression, and
   (c) isolating the fusion protein according to the invention from the host cells or the cell-free protein synthesis system.
In **an eleventh aspect,** the invention pertains to a use of the fusion protein according to the invention for increasing protein degradation of a target protein, preferably wherein the fusion protein comprises at least one antigen binding domain specifically binding to the target protein.
In **a twelfth aspect,** the invention pertains to a method for screening a candidate fusion protein for an improved effect on lysosome-dependent protein degradation of a target protein, the method comprising:
   (a) providing a candidate fusion protein according to the invention with at least one amino acid mutation in the C-terminal domain of a progranulin polypeptide or a variant thereof,
   (b) contacting the candidate fusion protein and a target protein under conditions enabling degradation of the target protein by lysosomes,
   (c) determining the amount or level of target protein degradation over time compared to a control or reference,
   wherein an increased target protein degradation compared to the control or reference indicates that the at least one amino acid mutation in the C-terminal domain of a progranulin polypeptide in the candidate fusion protein has an effect on lysosome-dependent protein degradation of a target protein.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

In **a first aspect,** the invention pertains to a truncated progranulin protein or a variant thereof, wherein said truncated progranulin protein is N-terminally truncated compared to amino acid sequence according to SEQ ID NO. 1.

The term "variant thereof" or "functional variant" of a protein means herein a variant protein, wherein the function, in relation to the invention defined as affinity and stability, is essentially retained. Thus, one or more amino acids that are not relevant for said function may have been exchanged. The term "variant thereof' or "functional variant" should also be understood to mean homologues from other mammals. Preferably the functional variants of the present invention retain the sortilin specific binding abilities of the herein described progranulin protein as shown in SEQ ID NO: 1, 2 and 3.

Preferred is the truncated progranulin protein according to the present invention, wherein said truncated progranulin protein has 400 - 500 amino acids truncated at its N-terminus as compared with wild type progranulin (SEQ ID NO. 1).

Preferred is the truncated progranulin protein according to the present invention, wherein said truncated progranulin comprises an amino acid sequence which is at least 80%, 85%, 90%, 95%, 98% or 99% identical to SEQ ID NO: 2 or 3, or comprises the amino acid sequence of SEQ ID NO: 2 or 3, or a functional fragment thereof.

In the context of the present invention, the term "functional fragment of the truncated progranulin" shall relate to a part of the protein or the amino acid sequence of the above protein that is truncated at the N- and/or C-terminus of the amino acid sequence, but still performs and/or supports its function as required/desired in the context of the present invention, namely - when expressed, translated and/or administered - may be oxidized, and binds as disclosed herein. Preferably the functional fragments of the present invention retain the sortilin specific binding abilities of the herein described progranulin protein as shown in SEQ ID NO: 1, 2 and 3.

As used herein, the terms "identical" or percent "identity", when used anywhere herein in the context of two or more nucleic acid or protein/polypeptide sequences, refer to two or more sequences or subsequences that are the same or have (or have at least) a specified percentage of amino acid residues or nucleotides that are the same (i.e., at, or at least, about 60% identity, preferably at, or at least, 65%, 70%, 75%, 80%, 85%, 86%, 87%, 88%, 89%, 90%, 91%, 92%, 93% or 94%, identity, and more preferably at, or at least, about 95%, 96%, 97%, 98%, 99%, or higher identity over a specified region - preferably over their full length sequences - , when compared and aligned for maximum correspondence over the comparison window or designated region) as measured using a sequence comparison algorithms, or by manual alignment and visual inspection (see, e.g., NCBI web site). In a particular embodiment, for example when comparing the protein or nucleic acid sequence of a truncated PGRN or variant thereof of the invention with wild-type protein, the percentage identity can be determined by the Blast searches or local alignments; in particular for amino acid identity, those using BLASTP 2.2.28+ with the following parameters: Matrix: BLOSUM62; Gap Penalties: Existence: 11, Extension: 1; Neighboring words threshold: 11; Window for multiple hits: 40.

Preferred is the truncated progranulin protein according to the present invention, wherein the protein is not a full length progranulin protein.

Preferred is the truncated progranulin protein according to the present invention, wherein the protein comprises at least granulin domains D and/or E; and preferably does not comprise one of domains G, F, B, A, C.

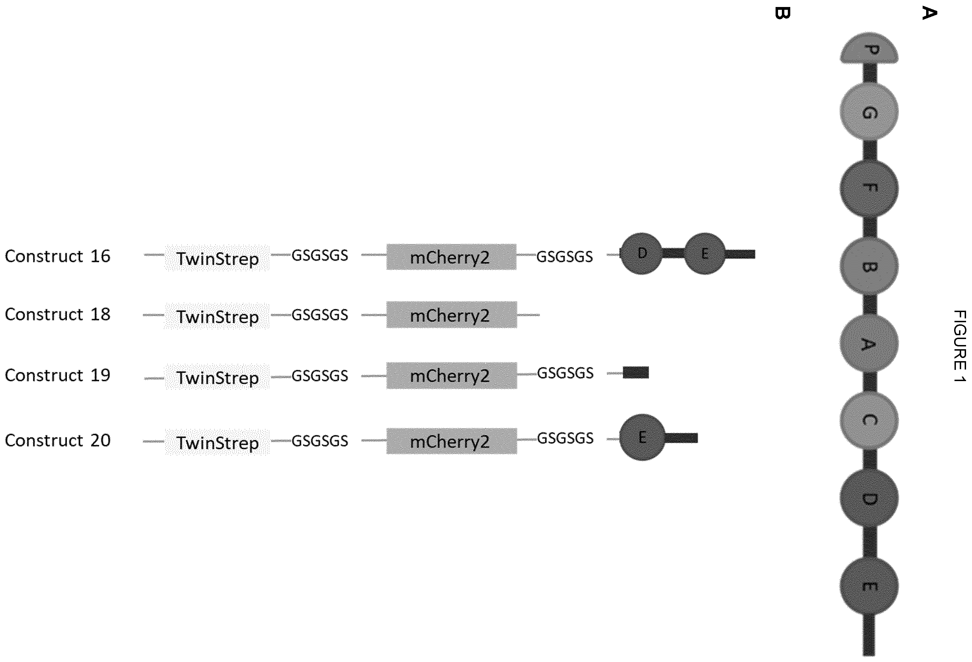

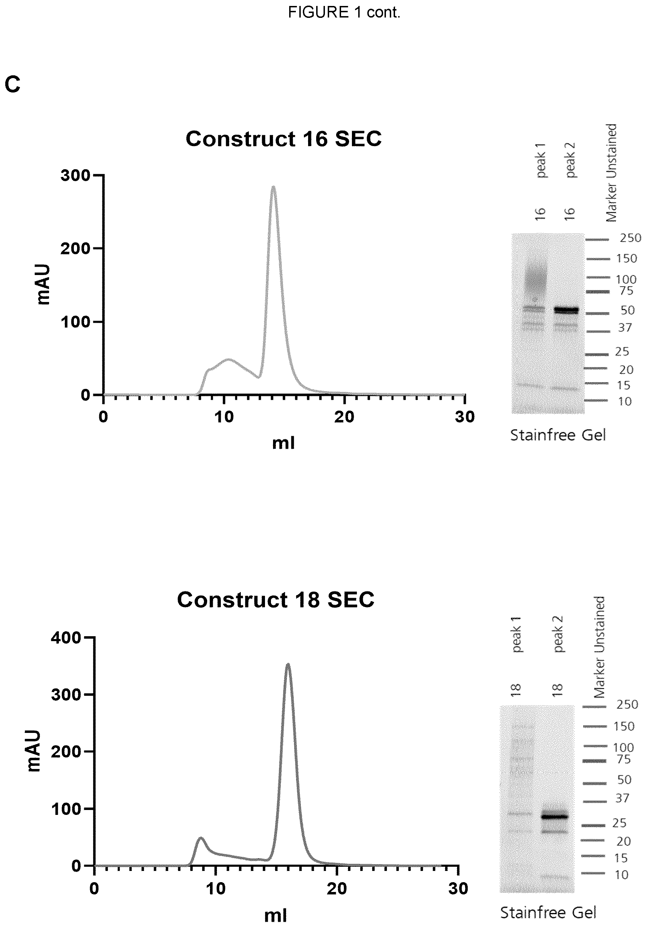

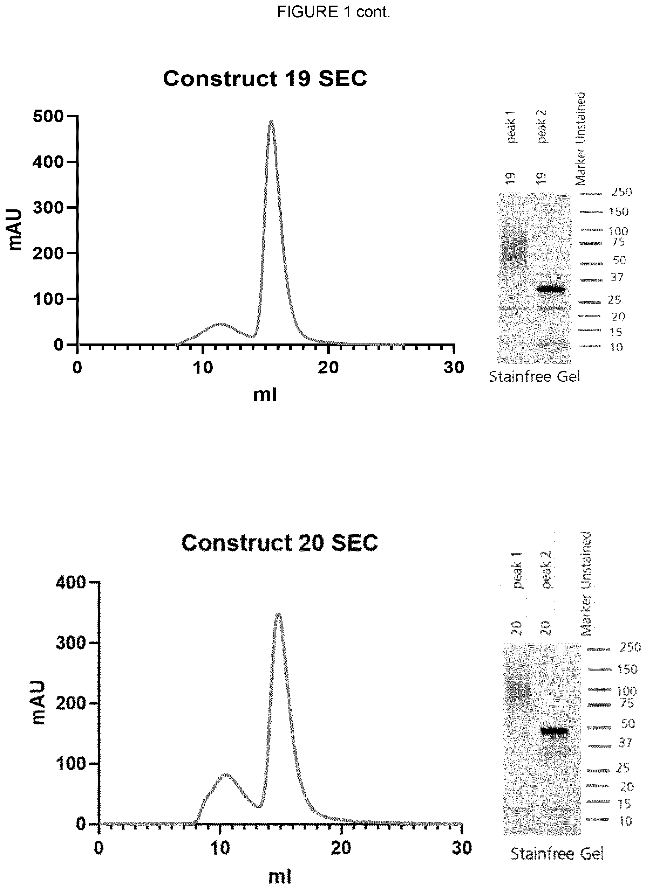

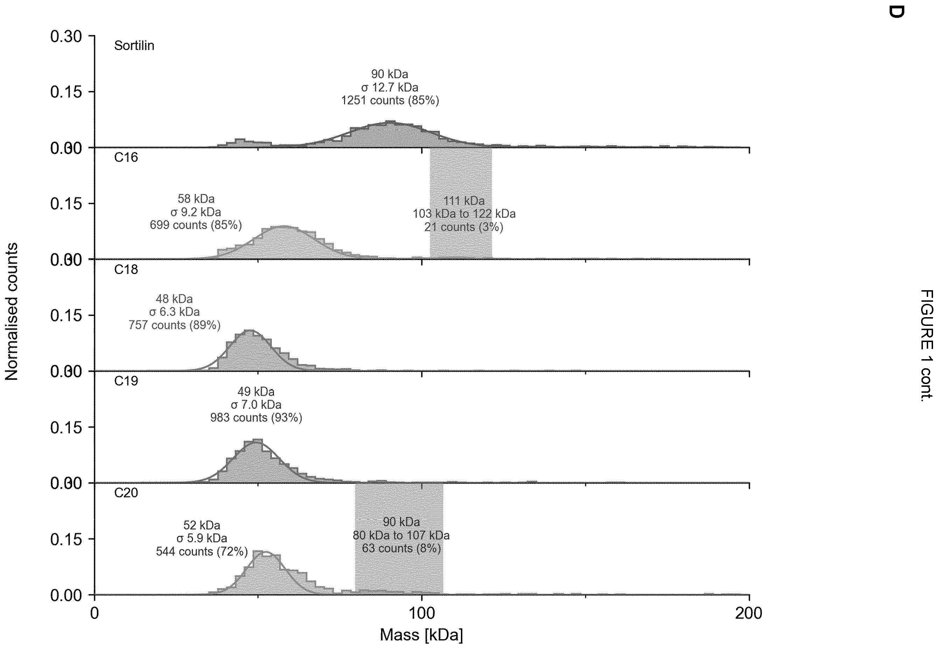

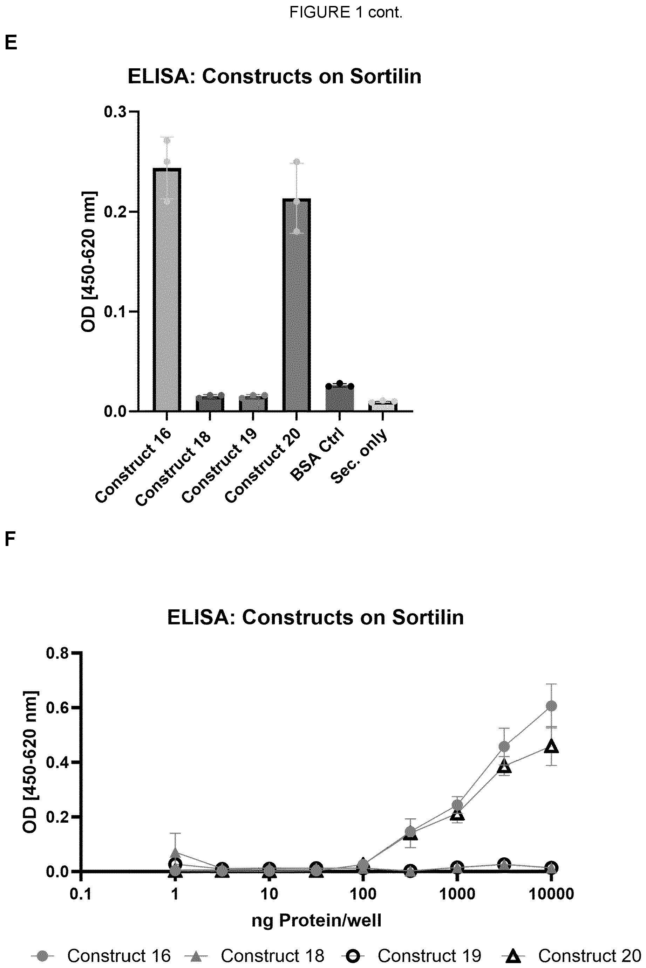

Preferred is the truncated progranulin protein according to the present invention, wherein the truncated progranulin protein does not comprise the domains G, F, B, A, and C (as shown in Figure 1).

In **a second aspect,** the invention pertains to a fusion protein comprising a truncated progranulin protein (or a progranulin fragment) fused to a non-progranulin protein, wherein the truncated progranulin protein or progranulin fragment is a protein according to the invention.

Preferred is the fusion protein according to the present invention, wherein the non-progranulin protein is fused to the truncated progranulin protein by a linker, such as a GS linker; preferably wherein (a) the N-terminal end of the truncated progranulin protein is fused to the C-terminus of the non-progranulin protein or (b) the C-terminal end of the truncated progranulin protein is fused to the N-terminus of the non-progranulin protein.

Preferred is the fusion protein according to the present invention, wherein the non-progranulin protein is selected from an antigen binding protein, an enzyme, a structural protein, a membrane anchor, a ligand binding domain or a receptor binding domain.

Another option is that a protein for use in a fusion of the invention is a synthetic protein such as a protein designed using generative artificial intelligence (Al). For example, methods for generating such synthetic proteins are described in WO/2024/151579 (incorporated by reference herein in its entirety).

Preferred is the fusion protein according to the present invention, wherein the non-progranulin protein is an antigen binding protein, preferably selected from an antibody, nanobody, T cell receptor, or any antigen binding fragments thereof.

In **a third aspect,** the invention pertains to a fusion protein comprising:
- an antigen binding region,
- optionally linked by a linker region with
- a progranulin domain comprising the sequence of the truncated progranulin protein or a variant thereof according to the invention,
wherein the progranulin domain specifically binds to sortilin.

In view of the results as obtained in the experiments as performed in the context of the present invention, the inventors identified fusion proteins that promote targeted lysosome-dependent protein degradation based on the interaction of the C- terminal domain of a progranulin polypeptide with sortilin.

Preferred is the fusion protein according to the present invention, wherein the antigen binding region is selected from the group consisting of an antibody, a minibody, a diabody, an antigen-binding fragment (Fab), a single-chain variable fragment (scFv), a heavy-chain only antibody, a single domain antibody (nanobody), a chimeric antibody, and a T-cell receptor antigen binding fragment.

These antigen binding regions are known to a person skilled in the art and are published in Berland et al, but not limited thereto (10).

As used herein, the term "linker" can be any fusion protein linker known to a person skilled in the art. The general properties of linkers are derived from naturally-occurring multidomain proteins, which are the foundation in linker design. Empirical linkers designed by persons skilled in the art are generally classified into three categories according to their structures: flexible linkers, rigid linkers, and *in vivo* cleavable linkers, but not limited thereto. Besides the basic role in linking the functional domains together (as in flexible and rigid linkers) or releasing free functional domain *in vivo* (as in in vivo cleavable linkers), linkers offer many other advantages for the production of fusion proteins, such as improving biological activity, increasing expression yield, and achieving desirable pharmacokinetic profiles.

The most commonly used flexible linkers have sequences consisting primarily of stretches of Gly and Ser residues ("GS" linker). An example of the most widely used flexible linker has the sequence of (Gly-Gly-Gly-Gly-Ser)ₙ. By adjusting the copy number "n", the length of this GS linker can be optimized to achieve appropriate separation of the functional domains, or to maintain necessary inter-domain interactions. In a preferred embodiment the flexible polypeptide linker is selected from the group consisting of (GGGGS)n, (G)n, and (GS)n, preferably GSGSGS, wherein n is a natural number between 1 and 4.

Other flexible linker known to a person skilled in the art are KESGSVSSEQLAQFRSLD and EGKSSGSGSESKST. There are applied for the construction of a bioactive scFv. The Gly and Ser residues in the linker were designed to provide flexibility, whereas Glu and Lys were added to improve the solubility. Further linker known to a person skilled in the art are published in Chen *et al,* but not limited thereto (11).

Preferred is the fusion protein according to the present invention, wherein the antigen-binding region is obtained from an antigen-binding region library.

As used herein, the term "antigen-binding region library" includes human naive library, immunized library from non-human animal and human, semi-synthetic library and synthetic library which are libraries known to those skilled in the art (Methods Mol Biol. 2002; 178: 87-100; J Immunol Methods. 2004 Jun; 289 (1-2): 65-80; and Expert Opin Biol Ther. 2007 May; 7(5): 763-79), but not limited thereto.

Preferred is the fusion protein according to the present invention, wherein the antigen binding region comprises
(a) a human FcRn-binding domain which has a human FcRn-binding activity at pH 5.8 to pH 6.0 and at pH 7.4, wherein the human FcRn-binding activity at pH 7.0 and at 25°C is stronger than KD 3.2 mM, and wherein the human FcRn-binding domain is an Fc domain resulting from substituting a different amino acid for at least one amino acid in the Fc domain of a human IgG1, IgG2, IgG3 or IgG4; and
(b) an antigen-binding domain which has a lower antigen-binding activity at pH 5.8 to pH 6.0 than at pH 7.4 and wherein the ratio of antigen-binding activity at pH 5.8 and at pH 7.4 is 2 or greater, 10 or greater or 40 or greater in the value of KD (at pH 5.8)/KD (at pH 7.4).

Known pH-dependent antigen-binding antibodies, which strongly bind to an antigen under the neutral conditions in plasma and dissociate from the antigen under acidic conditions in the endosome, can dissociate from the antigen in the endosome. When a pH-dependent antigen-binding antibody dissociates from the antigen, it is recycled to the plasma by FcRn. Therefore, it can bind to another antigen again. Thus, a single pH-dependent antigen-binding antibody can bind to a number of antigens repeatedly.

In eukaryotic cells the interior space of endosomes, lysosomes, and a variety of other intracellular organelles is maintained at a pH considerably more acidic than either cytosolic pH or extracellular pH. The pH value in the endosome is comparable to the lysosome. Therefore, by introducing a human FcRn-binding domain in the fusion protein described herein, additional to an antigen-binding domain, the fusion protein is recycled from the lysosome to the plasma by FcRn. This mechanism is well known to a person skilled in the art and disclosed for example in EP 3 702 368 B1, but not limited thereto.

Preferred is the fusion protein according to the present invention, wherein the fusion protein comprises a linker selected from the group consisting of flexible linkers, rigid linkers and in vivo cleavable linkers, preferably flexible linkers.

Preferred is the fusion protein according to the present invention, wherein the fusion protein further comprises a signal peptide comprising the amino acid sequence which is at least 98 % or 99 % identical to SEQ ID NO: 4.

In **a fourth aspect,** the invention pertains to a nucleic acid sequence encoding the truncated progranulin protein according to the invention or the fusion protein according to the invention.

In the fourth aspect the problem is solved by an isolated nucleic acid comprising a sequence coding for the truncated progranulin protein or a fusion protein as described herein before, or encoding for a functional fragment of the fusion protein as described herein before. The term "encoding" or more simply "coding" refers to the ability of a nucleotide sequence to code for one or more amino acids. The term does not require a start or stop codon. An amino acid sequence can be encoded in any one of six different reading frames provided by a polynucleotide sequence and its complement. An amino acid sequence can be encoded by desoxyribonucleic acid (DNA), ribonucleic acid (RNA), or artificially synthesized polymers similar to DNA or RNA.

In **a fifth aspect,** the invention pertains to a vector comprising the nucleic acid sequence according to the invention, preferably an expression vector, more preferably comprising a promoter sequence operably linked to the nucleic acid sequence according to the invention.

A "vector" may be any agent that is able to deliver or maintain a nucleic acid in a host cell and includes, for example, but is not limited to, plasmids (e.g., DNA plasmids), naked nucleic acids, viral vectors, viruses, nucleic acids complexed with one or more polypeptide or other molecules, as well as nucleic acids immobilized onto solid phase particles. Vectors are described in detail below. A vector can be useful as an agent for delivering or maintaining an exogenous gene and/or protein in a host cell. A vector may be capable of transducing, transfecting, or transforming a cell, thereby causing the cell to replicate or express nucleic acids and/or proteins other than those native to the cell or in a manner not native to the cell. The target cell may be a cell maintained under cell culture conditions or in other in vivo embodiments, being part of a living organism. A vector may include materials to aid in achieving entry of a nucleic acid into the cell, such as a viral particle, liposome, protein coating, or the like. Any method of transferring a nucleic acid into the cell may be used; unless otherwise indicated, the term vector does not imply any particular method of delivering a nucleic acid into a cell or imply that any particular cell type is the subject of transduction. The present invention is not limited to any specific vector for delivery or maintenance of any nucleic acid of the invention, including, e.g., a nucleic acid encoding the fusion protein of the invention or a fragment thereof.

Preferably the vector of the invention is an expression vector. The term "expression vector" typically refers to a nucleic acid construct or sequence, generated recombinantly or synthetically, with a series of specific nucleic acid elements that permit transcription of a particular nucleic acid in a host cell. The expression vector typically includes a nucleic acid to be transcribed - the fusion protein of the invention - operably linked to a promoter. The term "expression" includes any step involved in the production of the polypeptide including, but not limited to, transcription, post-transcriptional modification, translation, post-translational modification, and/or secretion. A preferred vector of the invention is a plant-specific, bacterial, yeast, insect, vertebrate, preferably mammalian, or a viral vector, preferably retroviral and adeno-associated viral vector. Preferred vectors of the invention are suitable for use in gene therapy, preferably gene therapy based on transformation of autologous adult stem cells.

In **a sixth aspect,** the invention pertains to a host cell comprising the truncated progranulin protein according to the invention, the fusion protein according to the invention, the nucleic acid sequence according to the invention, or the vector according to the invention, preferably a host cell having the nucleic acid sequence according to the invention integrated in its genome.

A "recombinant cell" or also referred to as "host cell" is any cell that is susceptible to transformation with a nucleic acid. Preferably the recombinant or host cell of the invention is a plant cell, bacterial cell, yeast cell, an insect cell or a vertebrate, preferably a mammalian cell. A preferred recombinant cell is selected from a cell suitable for recombinant expression of the fusion protein of the invention. Most preferred is a Chinese hamster ovary (CHO) cell.

In **a seventh aspect,** the invention pertains to a pharmaceutical composition comprising:
(i) the truncated progranulin protein according to the invention, the fusion protein according to the invention, the nucleic acid sequence according to the invention, the vector according to the invention, or the host cell according to the invention, and
(ii) a pharmaceutically acceptable carrier, stabilizer and/or excipient.

In the following the truncated progranulin, the fusion proteins, nucleic acids encoding the same, vectors and cells comprising these nucleic acids or mutated proteins, as well as pharmaceutical compositions thereof, will be referred to generally as "compounds of the invention".

As used herein the language "pharmaceutically acceptable carrier" is intended to include any and all solvents, solubilizers, fillers, stabilizers, binders, absorbents, bases, buffering agents, lubricants, controlled release vehicles, diluents, emulsifying agents, humectants, lubricants, dispersion media, coatings, antibacterial or antifungal agents, isotonic and absorption delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions. In certain embodiments, the pharmaceutically acceptable carrier comprises serum albumin.

The pharmaceutical composition of the invention is formulated to be compatible with its intended route of administration. Examples of routes of administration include parenteral, e.g., intrathecal, intracerebroventricular, intraparenchymal, intraarterial, intravenous, intradermal, subcutaneous, oral, transdermal (topical) and transmucosal administration. The term "intrathecal," as used herein, means introduced into or occurring in the space under the arachnoid membrane which covers the brain and spinal cord. The term "intracerebroventricular" refers to administration of a composition into the ventricular system of the brain, e.g., via injection, infusion, or implantation (for example, into a ventricle of the brain). As used herein, the term "intraparenchymal" can refer to an administration directly to brain tissue. In other instances, intraparenchymal administration may be directed to any brain region where delivery of one or more compounds of the invention is effective to mitigate or prevent one or more of disorders as described herein.

Solutions or suspensions used for parenteral, intradermal, or subcutaneous application can include the following components: a sterile diluent such as water for injection, saline solution, fixed oils, polyethylene glycols, glycerine; propylene glycol or other synthetic solvents; antibacterial agents such as benzyl alcohol or methyl parabens; antioxidants such as ascorbic acid or sodium bisulfate; chelating agents such as ethylenediaminetetraacetic acid; buffers such as acetates, citrates or phosphates and agents for the adjustment of tonicity such as sodium chloride, mannitol or dextrose. pH can be adjusted with acids or bases, such as hydrochloric acid or sodium hydroxide. The parenteral preparation can be enclosed in am-poules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, N.J.) or phosphate buffered saline (PBS). In all cases, the injectable composition should be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, mannitol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, and sodium chloride in the composition. Prolonged absorption of the injectable compositions can be brought about by including in the composition an agent which delays absorption, for example, aluminium monostearate and gelatine.

Sterile injectable solutions can be prepared by incorporating the active compound (e.g., a compound of the invention such as the fusion protein) in the required amount in an appropriate solvent with one or a combination of ingredients enumerated above, as required, followed by filtered sterilization. Generally, dispersions are prepared by incorporating the active compound of the invention into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatine capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatine; an excipient such as starch or lactose, a disintegrating agent such as alginic acid, Primogel, or corn starch; a lubricant such as magnesium stearate or stertes; a glidant such as colloidal silicon dioxide; a sweetening agent such as sucrose or saccharin; or a flavouring agent such as peppermint, methyl salicylate, or orange flavouring.

For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the pharmaceutical compositions are formulated into ointments, salves, gels, or creams as generally known in the art.

In certain embodiments, the pharmaceutical composition is formulated for sustained or con-trolled release of the active ingredient. Biodegradable, biocompatible polymers can be used, such as ethylene vinyl acetate, polyanhydrides, polyglycolic acid, collagen, serum albumin, polyorthoesters, polylactic acid, poly(butyl cyanoacrylate), and poly(lactic-co-glycolic) acid. Methods for preparation of such formulations will be apparent to those skilled in the art. The materials can also be obtained commercially from e.g. Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art.

It is especially advantageous to formulate oral or parenteral compositions in dosage unit form for ease of administration and uniformity of dosage. Dosage unit form as used herein includes physically discrete units suited as unitary dosages for the subject to be treated; each unit containing a predetermined quantity of active compound calculated to produce the de-sired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. The pharmaceutical compositions can be included in a container, pack, or dispenser together with instructions for administration.

In **an eighth aspect,** the invention pertains to a compound for use in the treatment of a disease, the compound being selected from the truncated progranulin protein according to the invention, the fusion protein according to the invention, the nucleic acid sequence according to the invention, the vector according to the invention, the host cell according to the invention, or the pharmaceutical composition according to the invention.

The disease can be any abnormal condition that adversely affects the structure or function of all or part of a human. Preferably the disease is selected from any disease which is caused by a target that cannot or can only be treated to a limited extent with the known pharmaceutical approaches such as with small molecules or common antibodies.

In **an alternative aspect,** the invention provides a method for treating a disease in a subject comprising administering to the subject a therapeutically effective amount of a compound of the invention to promote targeted lysosome-dependent protein degradation of the target protein that causes the disease.

The problem is furthermore solved in this eighth and alternative aspect by a medical use of the compounds of the invention in the treatment of a disease.

Compositions and methods of the present invention may be used to effectively treat individuals (patients or subjects) suffering from a disease.

The terms, "treat" or "treatment", as used herein, refers to amelioration of one or more symptoms associated with a specific disease, prevention or delay of the onset of one or more symptoms of a specific disease, and/or lessening of the severity or frequency of one or more symptoms of a specific disease.

In some embodiments, treatment refers to increased survival (e.g. survival time). For example, treatment can result in an increased life expectancy of a patient. In some embodiments, treatment according to the present invention results in an increased life expectancy of a patient by more than about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 100%, about 105%, about 110%, about 115%, about 120%, about 125%, about 130%, about 135%, about 140%, about 145%, about 150%, about 155%, about 160%, about 165%, about 170%, about 175%, about 180%, about 185%, about 190%, about 195%, about 200% or more, as compared to the average life expectancy of one or more control individuals with similar disease without treatment.

In some embodiments, treatment according to the present invention results in an increased life expectancy of a patient by more than about 6 month, about 7 months, about 8 months, about 9 months, about 10 months, about 11 months, about 12 months, about 2 years, about 3 years, about 4 years, about 5 years, about 6 years, about 7 years, about 8 years, about 9 years, about 10 years or more, as compared to the average life expectancy of one or more control individuals with similar disease without treatment.

In some embodiments, treatment according to the present invention results in long term survival of a patient. As used herein, the term "long term survival" refers to a survival time or life expectancy longer than about 40 years, 45 years, 50 years, 55 years, 60 years, or longer. The terms, "improve," "increase" or "reduce," as used herein, indicate values that are relative to a control. In some embodiments, a suitable control is a baseline measurement, such as a measurement in the same individual prior to initiation of the treatment described herein, or a measurement in a control individual (or multiple control individuals) in the absence of the treatment described herein. A "control individual" is an individual afflicted with the same form of a disease, who is about the same age and/or gender as the individual being treated (to ensure that the stages of the disease in the treated individual and the control individual(s) are comparable.

In some embodiments, the individual is an individual who has been recently diagnosed with the disease. Typically, early treatment (treatment commencing as soon as possible after diagnosis) is important to minimize the effects of the disease and to maximize the benefits of treatment.

A treatment according to the invention preferably comprises the administration of a therapeutically effective amount of the compound of the invention to a subject in need of the treatment.

Preferred embodiments pertain to the treatment which comprises the intravenous, intracerebral, intrathecal and/or intracerebroventricular injection or infusion of a therapeutically effective amount of the compound of the invention to a subject in need of the treatment.

The compounds of the invention for use in therapeutic treatments are administered to a patient suffering from a disorder as mentioned herein, in therapeutically effective doses. The therapeutic dose of compound of the invention can be administered as a single dose or divided doses given in certain intervals of time, for example as two, three, four or more daily doses. The therapeutic dose can also be administered as a continuous infusion into the cerebrospinal fluid (intrathecal or intracerebroventricular infusion) or blood (intravenous infusion) released from a pump, e.g., an implantable miniature pump.

In some embodiments, the fusion protein or functional fragments thereof, suitable for the present invention are produced in mammalian cells. Non-limiting examples of mammalian cells that may be used in accordance with the present invention include BALB/c mouse myeloma line (NSO/1, ECACC No:85110503); human retinoblasts (PER.C6, Cru-Cell, Leiden, The Netherlands); monkey kidney CV1 line transformed by SV40 (COS-7, ATCC CRL 1651); human embryonic kidney line (293 or 293 cells subcloned for growth in suspension culture, Graham et al, J. Gen Virol, 36:59,1977); human fibrosarcoma cell line (e.g., HT1080); baby hamster kidney cells (BHK, ATCC CCL 10); Chinese hamster ovary cells +/-DHFR (CHO, Urlaub and Chasin, Proc. Natl. Acad. Sci. USA, 77:4216, 1980); mouse Sertoli cells (TM4, Mather, Biol. Reprod., 23:243-251, 1980); monkey kidney cells (CV1 ATCC CCL 70); African green monkey kidney cells (VERO- 76, ATCC CRL-1 587); human cervical carcinoma cells (HeLa, ATCC CCL 2); canine kidney cells (MDCK, ATCC CCL 34); buffalo rat liver cells (BRL 3 A, ATCC CRL 1442); human lung cells (W138, ATCC CCL 75); human liver cells (Hep G2, HB 8065); mouse mammary tumour (MMT 060562, ATCC CCL51); TRI cells (Mather et al, Annals N.Y. Acad. Sci., 383:44-68, 1982); MRC 5 cells; FS4 cells; and a human hepatoma line (Hep G2).

In some embodiments, the fusion protein or functional fragments thereof comprise a moiety that binds to an antigen on the surface of target protein to facilitate lysosomal targeted degradation of the target protein. Such target protein can be any protein that causes a disease.

In some embodiments, a therapeutic protein includes additional to the C- terminal domain of a progranulin polypeptide or a variant thereof for the specific binding to sortilin for lysosomal targeted degradation, a membrane -penetrating peptide.

In **a nineth aspect,** the invention pertains to a kit comprising the truncated progranulin protein according to the invention, the fusion protein according to the invention, the nucleic acid sequence according to the invention, the vector according to the invention, the host cell according to the invention, or the pharmaceutical composition according to the invention.

In **a tenth aspect,** the invention pertains to a method for producing the truncated progranulin protein according to the invention or the fusion protein according to the invention, comprising the steps of:
(a) culturing and harvesting a host cell according to the invention under conditions suitable for protein expression; or
(b) adding the nucleic acid sequence according to the invention, or the vector according to the invention to a cell-free protein synthesis system under conditions suitable for protein expression, and
(c) isolating the fusion protein according to the invention from the host cells or the cell-free protein synthesis system.

In some embodiments, the truncated progranulin protein or the fusion protein or a functional fragment thereof, suitable for the present invention are produced in cell-free in vitro translation systems. Non-limiting examples of cell-free in vitro translation systems that may be used in accordance with the present invention include cell-free translation systems consisting of extracts from rabbit reticulocytes, wheat germ and *Escherichia coli.* All are prepared as crude extracts containing all the macromolecular components (70S or 80S ribosomes, tRNAs, aminoacyl-tRNA synthetases, initiation, elongation and termination factors, etc.) required for translation of exogenous RNA. To ensure efficient translation, each extract must be supplemented with amino acids, energy sources (ATP, GTP), energy regenerating systems (creatine phosphate and creatine phosphokinase for eukaryotic systems, and phosphoenol pyruvate and pyruvate kinase for the *E. coli* lysate), and other co-factors (Mg²⁺, K⁺, etc.). In a further embodiment, the fusion protein or the functional fragment thereof are produced in cell-free in vitro transcription translation systems. These systems, for example, consist of extracts from E. coli. In these systems DNA is used as a template and during transcription, the 5' end of the RNA becomes available for ribosomal binding and undergoes translation while its 3' end is still being transcribed.

In **an eleventh aspect,** the invention pertains to a use of the fusion protein according to the invention for increasing protein degradation of a target protein, preferably wherein the fusion protein comprises at least one antigen binding domain specifically binding to the target protein.

Preferred is the use of the fusion protein according to the present invention, comprising
(a) providing in a eukaryotic cell a target protein comprising the complementary antigen sequence to the antigen binding region of the fusion protein, and
(b) adding the fusion protein to the eukaryotic cell and incubating to enable the binding of the antigen binding region of the fusion protein to the antigen sequence of the target protein and the binding of the progranulin domain of the fusion protein to sortilin, under conditions enabling degradation of the target protein by lysosomes; and
(c) determining the amount of the target protein present in the cell relative to a control,
wherein, a reduced amount of the target protein relative to a control determines the fusion protein according to the invention as a protein that can be used for targeted protein degradation.

In **a twelfth aspect,** the invention pertains to a method for screening a candidate fusion protein for an improved effect on lysosome-dependent protein degradation of a target protein, the method comprising:
(a) providing a candidate fusion protein according to the invention with at least one amino acid mutation in the C-terminal domain of a progranulin polypeptide or a variant thereof,
(b) contacting the candidate fusion protein and a target protein under conditions enabling degradation of the target protein by lysosomes,
(c) determining the amount or level of target protein degradation over time compared to a control or reference,
wherein an increased target protein degradation compared to the control or reference indicates that the at least one amino acid mutation in the C-terminal domain of a progranulin polypeptide in the candidate fusion protein has an effect on lysosome-dependent protein degradation of a target protein.

The term "contacting" in the present invention means any interaction between the potentially candidate compound and a target protein.

The term "mutation" refers to, in the context of a polynucleotide, a modification to the polynucleotide sequence resulting in a change in the sequence of a polynucleotide with reference to a precursor polynucleotide sequence. A mutant polynucleotide sequence can refer to an alteration that does not change the encoded amino acid sequence, for example, with regard to codon optimization for expression purposes, or that modifies a codon in such a way as to result in a modification of the encoded amino acid sequence - such "silent" mutations are however in context of the present invention in preferred embodiments excluded. Mutations can be introduced into a polynucleotide through any number of methods known to those of ordinary skill in the art, including random mutagenesis, site-specific mutagenesis, oligonucleotide directed mutagenesis, gene shuffling, directed evolution techniques, combinatorial mutagenesis, site saturation mutagenesis among others.

"Mutation" or "mutated" means, in the context of a protein, a modification to the amino acid sequence resulting in a change in the sequence of a protein with reference to a precursor protein sequence. A mutation can refer to a substitution of one amino acid with another amino acid, an insertion or a deletion of one or more amino acid residues. Specifically, a mutation can also be the replacement of an amino acid with a non-natural amino acid, or with a chemicallymodified amino acid or like residues. A mutation can also be a truncation (e.g., a deletion or interruption) in a sequence or a subsequence from the precursor sequence. In context of the present invention a mutation is preferably an alteration that results into a removal of a certain amino acid side chain targeted by the mutation. A mutation may also be an addition of a subsequence (e.g., two or more amino acids in a stretch, which are inserted between two contiguous amino acids in a precursor protein sequence) within a protein, or at either terminal end of a protein, thereby increasing the length of (or elongating) the protein. A mutation can be made by modifying the DNA sequence corresponding to the precursor protein. Mutations can be introduced into a protein sequence by known methods in the art, for example, by creating synthetic DNA sequences that encode the mutation with reference to precursor proteins, or chemically altering the protein itself. A "mutant" as used herein is a protein comprising a mutation. For example, it is also possible to make a mutant by replacing a portion of progranulin of the inevntion with a wild-type sequence of human or non-human origin (preferably non-human vertebrates) that corresponds to such portion but includes a desired variation at a specific position that is naturally-occurring in the wild-type sequence.

Preferred is the method and use according to the present invention, wherein the determining of the amount of the target protein is performed through a method selected from the group consisting of western blot, capillary immunoassay, fluorescence- or luminescence-based reporter assays, mass spectrometry, ALPHA assay, and ELISA assay.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

In view of the above, it will be appreciated that the present invention also relates to the following itemised embodiments:
Item 1: A truncated progranulin protein or a variant thereof, wherein said truncated progranulin protein is N-terminally truncated compared to amino acid sequence according to SEQ ID NO. 1.
Item 2: The truncated progranulin protein of item 1, wherein said truncated progranulin protein has 400 - 500 amino acids truncated at its N-terminus as compared with wild type progranulin (SEQ ID NO. 1).
Item 3: The truncated progranulin protein of item 1 or 2, wherein said truncated progranulin comprises an amino acid sequence which is at least 80%, 85%, 90%, 95%, 98% or 99% identical to SEQ ID NO: 2 or 3, or comprises the amino acid sequence of SEQ ID NO: 2 or 3, or a functional fragment thereof.
Item 4: The truncated progranulin protein or variant thereof, of any one of items 1 to 3, wherein the protein is not a full length progranulin protein.
Item 5: The truncated progranulin protein or variant thereof, of any one of items 1 to 4, comprising at least granulin domains D and/or E; and preferably does not comprise one of domains G, F, B, A, C.
Item 6: The truncated progranulin protein or variant thereof of any one of items 1 to 5, wherein the truncated progranulin protein does not comprise the domains G, F, B, A, and C (as shown in Figure 1).
Item 7: A fusion protein comprising a truncated progranulin protein (or a progranulin fragment) fused to a non-progranulin protein, wherein the truncated progranulin protein or progranulin fragment is a protein recited in any one of items 1 to 6.
Item 8: The fusion protein of item 7, wherein the non-progranulin protein is fused to the truncated progranulin protein by a linker, such as a GS linker; preferably wherein (a) the N-terminal end of the truncated progranulin protein is fused to the C-terminus of the non-progranulin protein or (b) the C-terminal end of the truncated progranulin protein is fused to the N-terminus of the non-progranulin protein.
Item 9: The fusion protein of item 7 or 8, wherein the non-progranulin protein is selected from an antigen binding protein, an enzyme, a structural protein, a membrane anchor, a ligand binding domain or a receptor binding domain.
Item 10: The fusion protein of any one of items 7 to 9, wherein the non-progranulin protein is an antigen binding protein, preferably selected from an antibody, nanobody, T cell receptor, or any antigen binding fragments thereof.
Item 11: A fusion protein comprising
   - an antigen binding region,
   - optionally linked by a linker region with
   - a progranulin domain comprising the sequence of the truncated progranulin protein or a variant thereof of items 1 to 6,
   wherein the progranulin domain specifically binds to sortilin.
Item 12: The fusion protein of item 11, wherein the antigen binding region is selected from the group consisting of an antibody, a minibody, a diabody, an antigen-binding fragment (Fab), a single-chain variable fragment (scFv), a heavy-chain only antibody, a single domain antibody (nanobody), a chimeric antibody, and a T-cell receptor antigen binding fragment.
Item 13: The fusion protein of item 11 or 12, wherein the antigen-binding region is obtained from an antigen-binding region library.
Item 14: The fusion protein of any one of items 11 to 13, wherein the antigen binding region comprises
   (a) a human FcRn-binding domain which has a human FcRn-binding activity at pH 5.8 to pH 6.0 and at pH 7.4, wherein the human FcRn-binding activity at pH 7.0 and at 25°C is stronger than KD 3.2 mM, and wherein the human FcRn-binding domain is an Fc domain resulting from substituting a different amino acid for at least one amino acid in the Fc domain of a human IgG1, IgG2, IgG3 or IgG4; and
   (b) an antigen-binding domain which has a lower antigen-binding activity at pH 5.8 to pH 6.0 than at pH 7.4 and wherein the ratio of antigen-binding activity at pH 5.8 and at pH 7.4 is 2 or greater, 10 or greater or 40 or greater in the value of KD (at pH 5.8)/KD (at pH 7.4).
Item 15: The fusion protein of any one of items 11 to 14, wherein the fusion protein comprises a linker selected from the group consisting of flexible linkers, rigid linkers and *in vivo* cleavable linkers, preferably flexible linkers.
Item 16: The fusion protein of any one of items 11 to 15, wherein the fusion protein further comprises a signal peptide comprising the amino acid sequence which is at least 98 % or 99 % identical to SEQ ID NO: 4.
Item 17: A nucleic acid sequence encoding the truncated progranulin protein of any one of items 1 to 6 or the fusion protein of any one of items 7 to 16.
Item 18: A vector comprising the nucleic acid sequence of item 17, preferably an expression vector, more preferably comprising a promoter sequence operably linked to the nucleic acid sequence of item 17.
Item 19: A host cell comprising the truncated progranulin protein of any one of items 1 to 6, the fusion protein of any one of items 7 to 16, the nucleic acid sequence of item 17, or the vector of item 18 preferably a host cell having the nucleic acid sequence of item 17 integrated in its genome.
Item 20: A pharmaceutical composition comprising:
   (i) the truncated progranulin protein of any one of items 1 to 6, the fusion protein of any one of items 7 to 16, the nucleic acid sequence of item 17, or the vector of item 18, or the host cell of item 19, and
   (ii) a pharmaceutically acceptable carrier, stabilizer and/or excipient.
Item 21: A compound for use in the treatment of a disease, the compound being selected from the truncated progranulin protein of any one of items 1 to 6, the fusion protein of any one of items 7 to 16, the nucleic acid sequence of item 17, or the vector of item 18, or the host cell of item 19, the pharmaceutical composition of item 20.
Item 22: A kit comprising the truncated progranulin protein of any one of items 1 to 6, the fusion protein of any one of items 7 to 16, the nucleic acid sequence of item 17, or the vector of item 18, or the host cell of item 19, or the pharmaceutical composition of item 20.
Item 23: A method for producing the truncated progranulin protein of any one of items 1 to 6 or the fusion protein of any one of items 7 to 16, comprising the steps of:
   (d) culturing and harvesting a host cell of item 19 under conditions suitable for protein expression; or
   (e) adding the nucleic acid sequence of item 17, or the vector of item 18 to a cell-free protein synthesis system under conditions suitable for protein expression, and
   (f) isolating the truncated progranulin protein of any one of items 1 to 6 or the fusion protein of any one of items 7 to 16 from the host cells or the cell-free protein synthesis system.
Item 24: A use of the fusion protein of any one of items 7 to 16 for increasing protein degradation of a target protein, preferably wherein the fusion protein comprises at least one antigen binding domain specifically binding to the target protein.
Item 25: The use of item 24, comprising:
   (a) providing in a eukaryotic cell a target protein comprising the complementary antigen sequence to the antigen binding region of the fusion protein,
   (b) adding the fusion protein to the eukaryotic cell and incubating to enable the binding of the antigen binding region of the fusion protein to the antigen sequence of the target protein and the binding of the progranulin domain of the fusion protein to sortilin, under conditions enabling degradation of the target protein by lysosomes; and
   (c) determining the amount of the target protein present in the cell relative to a control,
   wherein, a reduced amount of the target protein relative to a control determines the fusion protein of any items 7 to 16 as a protein that can be used for targeted protein degradation.
Item 26: A method for screening a candidate fusion protein for an improved effect on lysosome-dependent protein degradation of a target protein, the method comprising:
   (d) providing a candidate fusion protein comprising the fusion protein of any one of items 7 to 16 with at least one amino acid mutation in the progranulin domain,
   (e) contacting the candidate fusion protein and a target protein under conditions enabling degradation of the target protein by lysosomes,
   (f) determining the amount or level of target protein degradation over time compared to a control or reference,
   wherein an increased target protein degradation compared to the control or reference indicates that the at least one amino acid mutation in the progranulin domain in the candidate fusion protein has an effect on lysosome-dependent protein degradation of a target protein.
Item 27: The method and use of any one of items 24 to 26, wherein the determining of the amount of the target protein is performed through a method selected from the group consisting of western blot, capillary immunoassay, fluorescence- or luminescence-based reporter assays, mass spectrometry, ALPHA assay, and ELISA assay.

### BRIEF DESCRIPTION OF THE FIGURES AND SEQUENCES

The figures show:
**Figure 1****:** shows the design and validation of sortilin-binding progranulin (PGRN) fusion protein variants. (A) Schematic overview of PGRN domain architecture. (B) Overview of the four constructs for the fusion protein variants C16, C18, C19, and C20. (C) Size exclusion chromatography (SEC) diagrams and western blot analysis for all four fusion protein variants. (D) Mass photometric analysis of soluble sortilin variant and C16, C18, C19, and C20. (E) Interaction analysis of C16, C18, C19, and C20 with sortilin via ELISA. As controls were used a BSA control (BSA Ctrl) and an anti-Strep-Mab HRP secondary antibody control (Sec. only). (F) Concentration-dependent binding analysis of fusion protein variants C16, C18, C19, and C20.

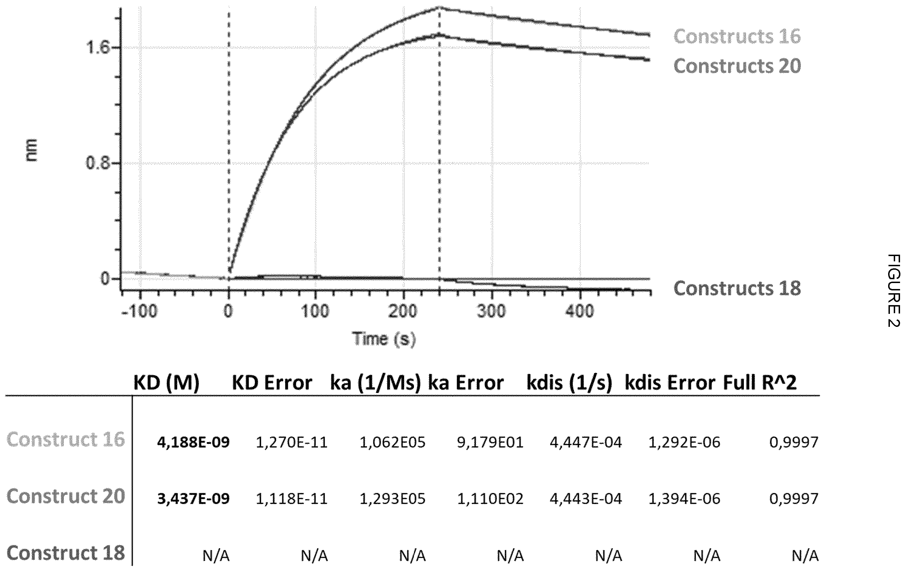

**Figure 2****:** shows the characterization of sortilin-binding properties of C16, C18, and C20 via sensorgrams of BLI measurements performed using a streptavidin sensor for immobilization of biotinylated extracellular domain of sortilin at 10 ng/µl. The sensorgrams show clear association and dissociation kinetics for C16 and C20 analytes, while no phase shift was observable for the C18 analyte run. And the overview of kinetic parameters for sortilin binding affinities shows for C16 and C20 a dissociation rate constant < 5 nM.

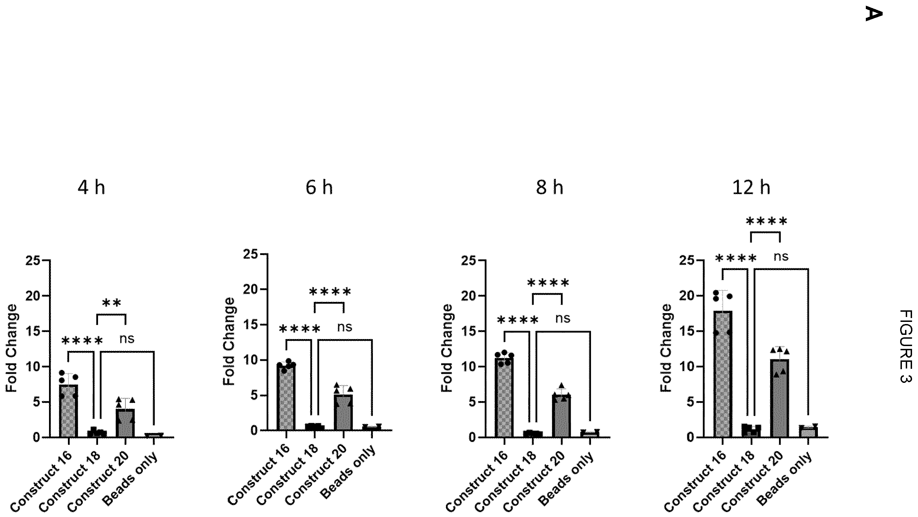

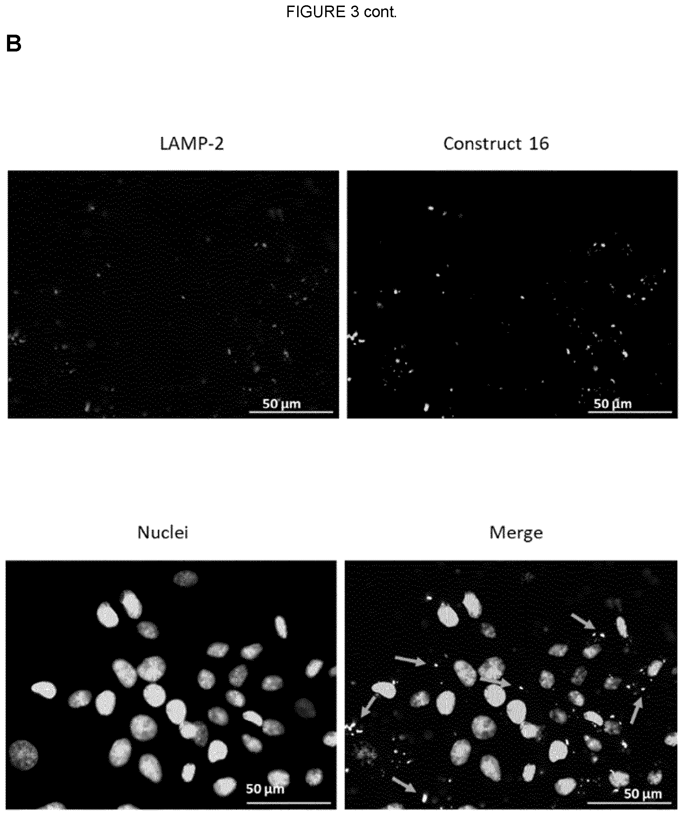

**Figure 3****:** shows uptake studies of the PGRN fusion proteins in cell-based assays. (A) Time course uptake study of fluorescently labeled fusion proteins C16, C18 and C20 by HepG2 cells via flow cytometry. (B) Immunofluorescent staining of HepG2 cells treated with C16 for 2 hours. Arrows in the merge picture show colocalization of C16 and LAMP-2.

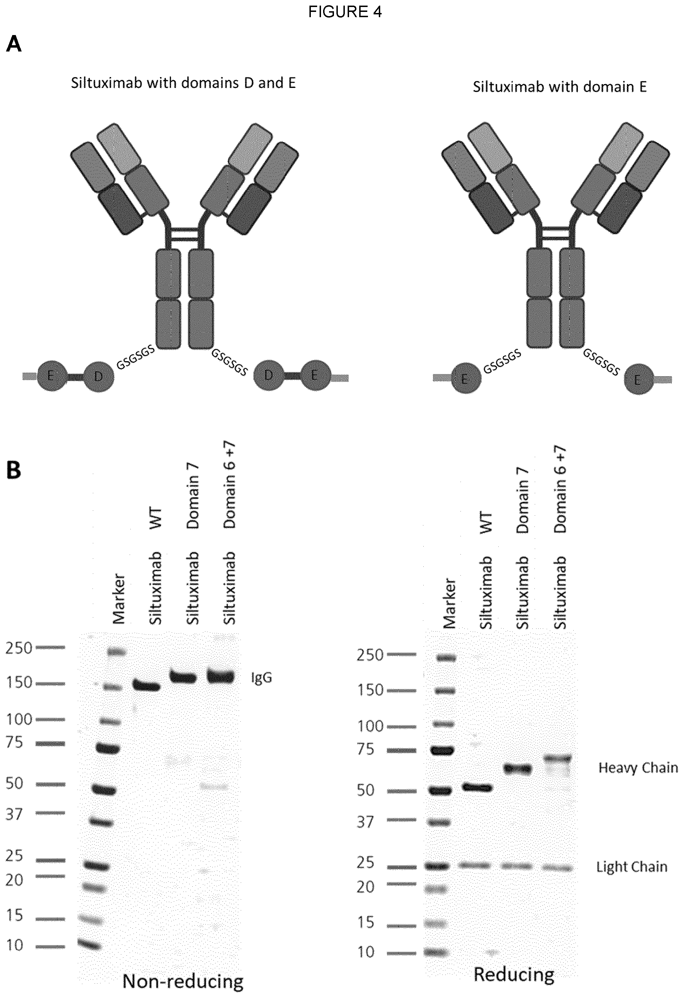

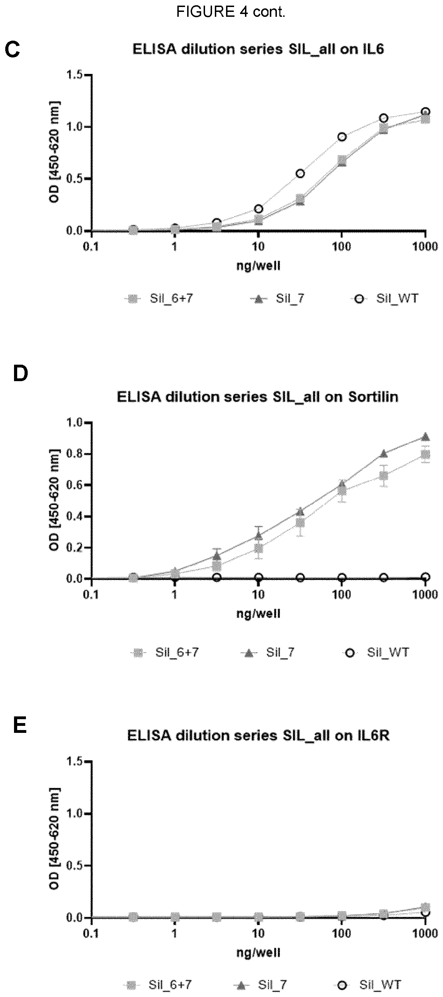

**Figure 4****:** shows the generation and validation of sortilin-binding siltuximab fusion proteins. (A) Overview of siltuximab-PGRN fusion proteins. Left fusion protein illustrates siltuximab with the domain D and E of PGRN (Sil_6+7) and the right fusion protein is siltuximab with domain E of PGRN (Sil_7). (B) Non-reducing (left) and reducing (right) SDS-PAGE analysis of siltuximab purified wildtype (Sil_WT), Sil_6+7 and Sil_7 fusion proteins. (C) IL6 binding studies of Sil_WT, Sil_6+7, and Sil_7 via ELISA (100 ng IL-6 coated/well). (D) Sortilin binding studies of Sil_WT, Sil_6+7, and Sil_7 via ELISA (100 ng extracellular domain of sortilin coated/well). (E) IL6R binding studies of Sil_WT, Sil_6+7, and Sil_7 via ELISA (100 ng IL6R coated/well).

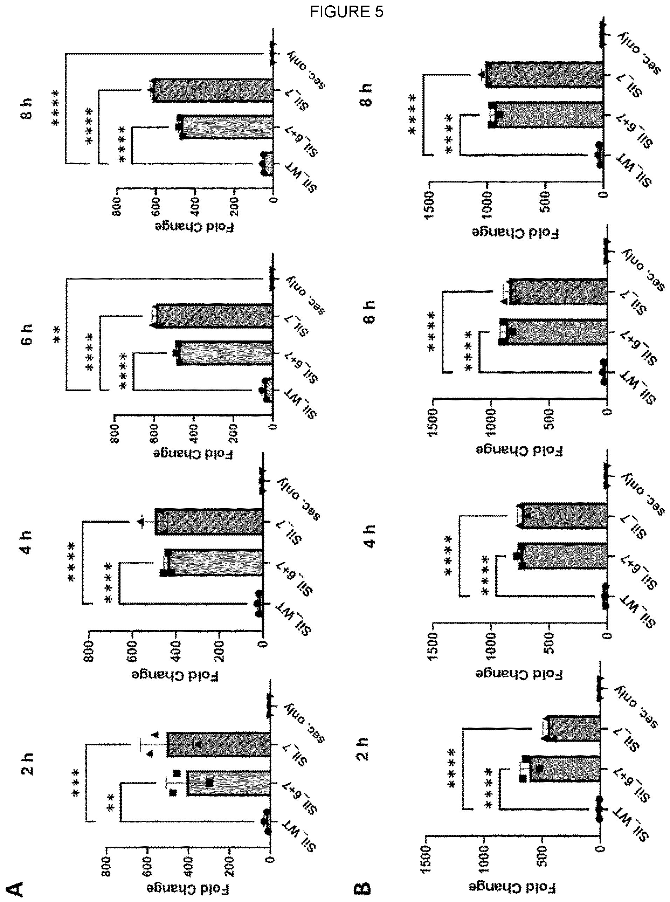

**Figure 5****:** shows cell uptake studies of the PGRN-siltuximab fusion proteins. (A) and (B) Fold change of uptake in HepG2 and SK-Hep1 cells was calculated based on the MFI of unstained cells. A significant fold change of MFI was detected for Sil_6+7 and Sil_7, MFI levels of Sil_WT and Sec.only remain at the level of baseline fluorescence of the cell lines over the time course of the experiment (****).

The sequences show:

### SEQ ID NOs. 1 to 15

**SEQ ID NO. 1:** PGRN
   >sp|P28799|GRN_HUMAN Progranulin OS=Homo sapiens OX=9606 GN=GRN PE=1 SV=2 Domain 1: Segment 58-113, Domain 2: Segment 123-179, Domain 3: Segment 206-261, Domain 4: Segment 281-336, Domain 5: Segment 364-417, Domain 6: Segment 442-496, Domain 7: Segment 518-573, unstructured C-terminus of PGRN 574-593.
**SEQ ID NO. 2:** Domain 6, 7 and unstructured C-terminus of PGRN 
IGCDQHTSCPVGQTCCPSLGGSWACCQLPHAVCCEDRQHCCPAGYTCNVKARSCEKEVVSA
QPATFLARSPHVGVKDVECGEGHFCHDNQTCCRDNRQGWACCPYRQGVCCADRRHCCPAG
FRCAARGTKCLRREAPRWDAPLRDPALRQLL
**SEQ ID NO. 3:** Domain 7 and unstructured C-terminus of PGRN 
DVECGEGHFCHDNQTCCRDNRQGWACCPYRQGVCCADRRHCCPAGFRCAARGTKCLRRE
APRWDAPLRDPALRQLL
**SEQ ID NO. 4:** Signal peptide
   MYRMQLLSCIALSLALVTN
**SEQ ID NO. 5:** TwinStrep-Tag
   WSHPQFEKGGGSGGGSGGSAWSHPQFEKDI
**SEQ ID NO. 6:** Linker
   GSGSGS
**SEQ ID NO. 7:** mCherry (Mock) 
VSKGEEDNMAIIKEFMRFKVHMEGSVNGHEFEIEGEGEGRPYEGTQTAKLKVTKGGPLPFAWD
ILSPQFMYGSKAYVKHPADIPDYLKLSFPEGFNWERVMNFEDGGVVTVTQDSSLQDGEFIYKV
KLRGTNFPSDGPVMQCRTMGWEASTERMYPEDGALKGEIKQRLKLKDGGHYDAEVKTTYKA
KKPVQLPGAYNVDIKLDILSHNEDYTIVEQYERAEGRHSTGGMDELYKDI
**SEQ ID NO. 8:** Construct 16 (C16) (Figure 1 B) 
MYRMQLLSCIALSLALVTNSWSHPQFEKGGGSGGGSGGSAWSHPQFEKDIGSGSGSVSKGE
EDNMAIIKEFMRFKVHMEGSVNGHEFEIEGEGEGRPYEGTQTAKLKVTKGGPLPFAWDILSPQ
FMYGSKAYVKHPADIPDYLKLSFPEGFNWERVMNFEDGGVVTVTQDSSLQDGEFIYKVKLRGT
NFPSDGPVMQCRTMGWEASTERMYPEDGALKGEIKQRLKLKDGGHYDAEVKTTYKAKKPVQ
LPGAYNVDIKLDILSHNEDYTIVEQYERAEGRHSTGGMDELYKDIGSGSGSIGCDQHTSCPVGQ
TCCPSLGGSWACCQLPHAVCCEDRQHCCPAGYTCNVKARSCEKEVVSAQPATFLARSPHVG
 
VKDVECGEGHFCHDNQTCCRDNRQGWACCPYRQGVCCADRRHCCPAGFRCAARGTKCLR
REAPRWDAPLRDPALRQLL
**SEQ ID NO. 9:** Construct 18 (C18) (Figure 1 B) 
MYRMQLLSCIALSLALVTNSWSHPQFEKGGGSGGGSGGSAWSHPQFEKDIGSGSGSVSKGE
EDNMAIIKEFMRFKVHMEGSVNGHEFEIEGEGEGRPYEGTQTAKLKVTKGGPLPFAWDILSPQ
FMYGSKAYVKHPADIPDYLKLSFPEGFNWERVMNFEDGGVVTVTQDSSLQDGEFIYKVKLRGT
NFPSDGPVMQCRTMGWEASTERMYPEDGALKGEIKQRLKLKDGGHYDAEVKTTYKAKKPVQ
LPGAYNVDIKLDILSHNEDYTIVEQYERAEGRHSTGGMDELYKDI
**SEQ ID NO. 10:** Construct 19 (C19) (Figure 1 B) 
MYRMQLLSCIALSLALVTNSWSHPQFEKGGGSGGGSGGSAWSHPQFEKDIGSGSGSVSKGE
EDNMAIIKEFMRFKVHMEGSVNGHEFEIEGEGEGRPYEGTQTAKLKVTKGGPLPFAWDILSPQ
FMYGSKAYVKHPADIPDYLKLSFPEGFNWERVMNFEDGGVVTVTQDSSLQDGEFIYKVKLRGT
NFPSDGPVMQCRTMGWEASTERMYPEDGALKGEIKQRLKLKDGGHYDAEVKTTYKAKKPVQ
LPGAYNVDIKLDILSHNEDYTIVEQYERAEGRHSTGGMDELYKDIGSGSGSRREAPRWDAPLR
DPALRQLL
**SEQ ID NO. 11:** Construct 20 (C20) (Figure 1 B) 
MYRMQLLSCIALSLALVTNSWSHPQFEKGGGSGGGSGGSAWSHPQFEKDIGSGSGSVSKGE
EDNMAIIKEFMRFKVHMEGSVNGHEFEIEGEGEGRPYEGTQTAKLKVTKGGPLPFAWDILSPQ
FMYGSKAYVKHPADIPDYLKLSFPEGFNWERVMNFEDGGVVTVTQDSSLQDGEFIYKVKLRGT
NFPSDGPVMQCRTMGWEASTERMYPEDGALKGEIKQRLKLKDGGHYDAEVKTTYKAKKPVQ
LPGAYNVDIKLDILSHNEDYTIVEQYERAEGRHSTGGMDELYKDIGSGSGSDVECGEGHFCHD
NQTCCRDNRQGWACCPYRQGVCCADRRHCCPAGFRCAARGTKCLRREAPRWDAPLRDPAL
RQLL
**SEQ ID NO. 12:** Siltuximab (DrugBank ID DB09036) Heavy Chain Sequence 
EVQLVESGGKLLKPGGSLKLSCAASGFTFSSFAMSWFRQSPEKRLEWVAEISSGGSYTYYPDT
VTGRFTISRDNAKNTLYLEMSSLRSEDTAMYYCARGLWGYYALDYWGQGTSVTVSSASTKGP
SVFPLAPSSKSTSGGTAALGCLVKDYFPEPVTVSWNSGALTSGVHTFPAVLQSSGLYSLSSVVT
VPSSSLGTQTYICNVNHKPSNTKVDKKVEPKSCDKTHTCPPCPAPELLGGPSVFLFPPKPKDTL
MISRTPEVTCVVVDVSHEDPEVKFNWYVDGVEVHNAKTKPREEQYNSTYRVVSVLTVLHQDW
LNGKEYKCKVSNKALPAPIEKTISKAKGQPREPQVYTLPPSRDELTKNQVSLTCLVKGFYPSDIA
VEWESNGQPENNYKTTPPVLDSDGSFFLYSKLTVDKSRWQQGNVFSCSVMHEALHNHYTQK
SLSLSPGK
**SEQ ID NO. 13:** Siltuximab (DrugBank ID DB09036) Light Chain Sequence QIVLIQSPAIMSASPGEKVTMTCSASSSVSYMYWYQQKPGSSPRLLIYDTSNLASGVPVRFSGS 
GSGTSYSLTISRMEAEDAATYYCQQWSGYPYTFGGGTKLEIKRTVAAPSVFIFPPSDEQLKSGT
ASVVCLLNNFYPREAKVQWKVDNALQSGNSQESVTEQDSKDSTYSLSSTLTLSKADYEKHKVY
ACEVTHQGLSSPVTKSFNRGEC
SEQ ID NO. 14: Signal peptide_Siltuximab heavy chain_Linker_Domain 6, 7 and unstructured C-terminus of PGRN_fusion protein 
MYRMQLLSCIALSLALVTNSEVQLVESGGKLLKPGGSLKLSCAASGFTFSSFAMSWFRQSPEK
RLEWVAEISSGGSYTYYPDTVTGRFTISRDNAKNTLYLEMSSLRSEDTAMYYCARGLWGYYAL
DYWGQGTSVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKTHTCPPCP
APELLGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLPPSRDE
LTKNQVSLTCLVKGFYPSDIAVEWESNGQPENNYKTTPPVLDSDGSFFLYSKLTVDKSRWQQG
NVFSCSVMHEALHNHYTQKSLSLSPGKGSGSGSIGCDQHTSCPVGQTCCPSLGGSWACCQL
PHAVCCEDRQHCCPAGYTCNVKARSCEKEVVSAQPATFLARSPHVGVKDVECGEGHFCHDN
QTCCRDNRQGWACCPYRQGVCCADRRHCCPAGFRCAARGTKCLRREAPRWDAPLRDPALR
QLL
SEQ ID NO. 15: Signal peptide_Siltuximab heavy chain_Linker_Domain 7 and unstructured C-terminus of PGRN_fusion protein

MYRMQLLSCIALSLALVTNSEVQLVESGGKLLKPGGSLKLSCAASGFTFSSFAMSWFRQSPEK
RLEWVAEISSGGSYTYYPDTVTGRFTISRDNAKNTLYLEMSSLRSEDTAMYYCARGLWGYYAL
DYWGQGTSVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKTHTCPPCP
APELLGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLPPSRDE
LTKNQVSLTCLVKGFYPSDIAVEWESNGQPENNYKTTPPVLDSDGSFFLYSKLTVDKSRWQQG
NVFSCSVMHEALHNHYTQKSLSLSPGKGSGSGSDVECGEGHFCHDNQTCCRDNRQGWACC
PYRQGVCCADRRHCCPAGFRCAARGTKCLRREAPRWDAPLRDPALRQLL

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Design and validation of sortilin-binding progranulin (PGRN) fusion protein variants.

In the context of the present invention, the inventors constructed fusion protein variants to study the effect of the fusion proteins on lysosome-dependent protein degradation of a target protein. Progranulin (PGRN) is a precursor protein (68 kDa) and becomes maturated by proteolytic cleavage into 7 individual granulin domains of ca. 6kDa within the lysosome. The schematic overview of the PGRN domain architecture includes the domains G(1), F(2), B(3), A(4), C(5), D(6), and E(7) as shown in Figure 1 A. PGRN is a ligand of Sortilin and is trafficked to the lysosome upon receptor engagement for proprotein maturation.

Based on this knowledge the inventors conducted a research study of possible sortilin-binding progranulin (PGRN) fusion protein variants that induce lysosome-dependent protein degradation of a target protein. The constructs for the fusion protein variants are shown in Figure 1 B. All constructs contain an N-terminal Twin-Strep tag for purification followed by a mock protein that is followed by a flexible glycine-serine linker (GS). Construct 16 contains domain D and E as well as the unstructured C-terminus of PGRN. Construct 18 is the negative control with no PGRN domains. Construct 19 contains only the unstructured C-terminus of PGRN. Construct 20 contains the terminal E domain as well as the unstructured C-terminus of PGRN.

All fusion protein variants C16, C18, C19, and C20 were produced in Expi293 cells, purified via StreptactinXT-based affinity chromatography and polished via preparative size exclusion chromatography (SEC). Target constructs containing samples were pooled for downstream analysis and subsequent experiments. Figure 1 C shows the purified fusion proteins on a western blot and SEC of every fusion protein.

The mass histograms of a soluble sortilin variant and C16, C18, C19, C20 show homogenous samples with respective molecular masses corresponding to the size of the tested molecules. As shown in Figure 1 D sortilin (first row) is 90 kDa in size, C16 (second row) is ca. 58 kDa, C18 (third row) is ca. 48 kDa, C19 (fourth row) is ca. 49 kDa and C20 (fourth row) is ca. 52 kDa.

ELISA was performed by coating recombinantly produced and purified extracellular domain of sortilin (100 ng/well) and using 1 µg of the respective construct variants per well. Construct 16 and 20 show discernable binding, while construct 18 without a PGRN domain and construct 19 only with the unstructured C-terminus of PGRN behave similar to the BSA control (BSA Ctrl) and the anti-Strep-Mab HRP secondary antibody control (Sec. only). ELISA data are presented as mean values ± s.d. (n = 3 assay technical replicates) (Figure 1 E).

ELISA was performed by coating the recombinantly produced and purified extracellular domain of sortilin (100 ng/well) and using a range of concentrations from 1 to 10,000 ng of the respective construct variants per well. Our data demonstrate a concentration dependent binding of construct 16 and 20 (light orange and pink) to sortilin. Construct 18 and 19 did not show binding (green and dark orange) to sortilin at any of the tested concentrations. Given that the unstructured C-terminal stretch of PGRN was not sufficient for sortilin binding, the inventors excluded construct 19 in subsequent experiments. ELISA data are presented as mean values ± s.d. (n = 3 assay technical replicates) (Figure 1 F).

### Example 2: Characterization of sortilin-binding properties of C16, C18, and C20.

The Bio-Layer Interferometry (BLI) is an optical technique for measuring macromolecular interactions by analyzing interference patterns of white light reflected from the surface of a biosensor tip. BLI measurements were performed using a Streptavidin sensor for immobilization of biotinylated extracellular domain of sortilin at 10 ng/µl. The sensorgrams show clear association and dissociation kinetics for C16 and C20 analytes, while no phase shift was observable for the C18 analyte run. Shown sensorgrams are corrected for low level non-specific binding to the sensor surface. A table with the kinetic parameters for sortilin binding of the three tested constructs shows that C16 and C20 both have a dissociation rate constant < 5 nM (Figure 2).

### Example 3: Uptake studies of the PGRN fusion proteins in cell-based assays.

Cells were serum starved 2 hours prior to the start of the experiment and placed on ice to synchronize their metabolism. A dose of 1.5 µg/well of StrepTactin DY649 labeled construct was used for each respective variant. Fold change was calculated based on the MFI of unstained cells. StrepTactin DY649 beads were used as probe control. Ordinary one-way ANOVA, Dunnett's multiple comparisons test with p < 0.1 (*), < 0.01(**), < 0.001 (***) and < 0.0001 (****). Data are derived as the mean of three independent experiments ± s.d (Figure 3).

Cells were fixed and permeabilized with 4% PFA and Triton X-100. Nuclei were stained with Hoechst (white) and the lysosome with a LAMP-2 (red) marker. Blue arrows in the merge show colocalization of construct C16 and LAMP-2 (yellow) (Figure 3).

### Example 4: Generation and validation of sortilin-binding siltuximab fusion proteins.

Sil_6+7 was generated by linking domains 6 and 7 (D and E) of PGRN to the C-terminus of a heavy chain via a flexible glycine-serine linker (GS), while Sil_7 was generated by linking only domain 7 (E) of PGRN (including the C-terminal stretch) to the C-terminus of a heavy chain (Figure 4 A).

Sil_6+7 and Sil_7 siltuximab antibody variants were analyzed under non-reducing (left) and reducing (right) SDS-PAGE analysis of Protein A purified wildtype as shown in Figure 4 B. Under non-reducing conditions siltuximab WT runs at about 150 kDa as expected for a typical IgG molecule. Siltuximab_7 migrates slightly higher with about 170 kDa while another size shift can be seen for Sil_6+7 at about 180 kDa. For reducing conditions all light chains migrate at 25 kDa, while for the heavy chain fragment a noticeable increase in size as a result of PGRN domain fusions is observable as compared to the wildtype heavy chain band.

Additionally, IL6 binding studies of Sil_WT, Sil_6+7, and Sil_7 were performed via ELISA (100 ng IL-6 coated/well). Binding of all three antibody variants to IL6 was similar across a concentration range from 0.1 ng/well to 1,000 ng/well (Figure 4 C). Furthermore, sortilin binding studies of Sil_WT, Sil_6+7, and Sil_7 were performed via ELISA (100 ng extracellular domain of Sortilin coated/well). While Sil_6+7 and Sil_7 bind sortilin in a concentration-dependent manner, Sil_WT (which lacks PGRN domain fusions) shows no specific interaction to sortilin at any of the tested concentrations (Figure 4 D).

Moreover, IL6R binding studies of Sil_WT, Sil_6+7, and Sil_7 were performed via ELISA (100 ng IL6R coated/well). This control dataset shows that none of the three siltuximab variants binds to IL6R. This setup was used as a control for unspecific binding. Overall, the ELISA experiments confirmed the specificity of the siltuximab-PGRN fusion proteins to both their antigen (IL6) and sortilin. ELISA data are presented as mean values ± s.d. (n = 3 assay technical replicates) (Figure 4 D).

### Example 5: Cell uptake studies of the PGRN-siltuximab fusion proteins.

Cells were serum starved 2 hours prior to the start of the experiment and placed on ice to synchronize their metabolism. For visualization, each respective siltuximab variant was labeled with goat anti-human F(ab')2 AF488 probe and analyzed 2, 4, 6, and 8 hours after exposure to cell lines via media. A final concentration of 100 nM was used in all assay conditions and for all tested siltuximab variants. Flow cytometry histograms of uptake into HepG2 cells and SK-Hep1 cells after 2, 4, 6, and 8-hours incubation with Sil_WT (first row), Sil_6+7 (second row), Sil_7 (third row), and an anti-strep-Mab HRP secondary antibody control (Sec.only) (Fourth row). Both fusion protein variants carrying the PGRN domains (Sil_6+7 and Sil_7) show a strong shift towards higher fluorescent intensities in both cell lines, while treatment with the wildtype variant of siltuximab (Sil_WT), as well as the anti-strep-Mab HRP secondary antibody control (Sec.only) does not induce any increase in fluorescence.

For both variants carrying the PGRN domains (Sil_6+7 (second row) and Sil_7 (third row)) the inventors observed a strong shift towards higher fluorescent intensities in both cell lines, while treatment with the wildtype variant of siltuximab (Sil_WT) (first row), as well as the secondary probe only (sec. only) (fourth row) does not induce any increase in fluorescence in the histograms (data not shown).

Fold change of uptake in HepG2 and SK-Hep1 cells was calculated based on the MFI of unstained cells. Herein, a similar pattern of uptake differences is noticeable as shown in the histograms. While a significant fold change of MFI was detected for Sil_6+7 and Sil_7, MFI levels of Sil_WT, and the secondary probe (sec. only) remain at the level of baseline fluorescence of the cell lines over the time course of the experiment. (****). Data are derived as the mean of three independent experiments ± s.d (Figure 5 A, B).

### REFERENCES

The references as cited:
1. Dang, C. V.; Reddy, E. P.; Shokat, K. M.; Soucek, L. Drugging the "undruggable" Cancer Targets. Nat. Rev. Cancer 2017, 17 (8), 502-508.
2. Spradlin JN, Zhang E, Nomura DK (2021). Reimagining Druggability Using Chemoproteomic Platforms. Acc Chem Res. 6;54(7):1801-1813. doi: 10.1021/acs.accounts.1c00065. Epub 2021 Mar 18. PMID: 33733731.
3. Sun X, Gao H, Yang Y, He M, Wu Y, Song Y, Tong Y, Rao Y (2019). PROTACs: great opportunities for academia and industry. In: Signal transduction and targeted therapy 4, S. 64. DOI: 10.1038/s41392-019-0101-6.
4. Toure, M. & Crews, C. M. Small-molecule PROTACS: new approaches to protein degradation. Angew. Chem. Int Ed. Engl. 55, 1966-1973 (2016).
5. Hu F, et al. Sortilin-mediated endocytosis determines levels of the frontotemporal dementia protein, progranulin. Neuron. 2010 Nov 18;68(4):654-67. doi: 10.1016/j.neuron.2010.09.034. PMID: 21092856; PMCID: PMC2990962.
6. Zhou X., et al. Impaired prosaposin lysosomal trafficking in frontotemporal lobar degeneration due to progranulin mutations. Nat Commun 8, 15277 (2017). https://doi.org/10.1038/ncomms15277.
7. Zheng Y, et al. C-terminus of progranulin interacts with the beta-propeller region of sortilin to regulate progranulin trafficking. PLoS One. 2011;6(6):e21023. doi: 10.1371/journal.pone.0021023. Epub 2011 Jun 15. PMID: 21698296; PMCID: PMC3115958.
8. De Muynck L, et al. The neurotrophic properties of progranulin depend on the granulin E domain but do not require sortilin binding. Neurobiol Aging. 2013 Nov;34(11):2541-7. doi: 10.1016/j.neurobiolaging.2013.04.022. Epub 2013 May 24. PMID: 23706646.
9. Logan T, et al. Rescue of a lysosomal storage disorder caused by Grn loss of function with a brain penetrant progranulin biologic. Cell. 2021 Sep 2;184(18):4651-4668.e25. doi: 10.1016/j.cell.2021.08.002. Epub 2021 Aug 26. Erratum in: Cell. Mar 14;187(6):1565-1566. doi: 10.1016/j.cell.2024.02.015. PMID: 34450028; PMCID: PMC8489356.
10. Berland, L.; Kim, L.; Abousaway, O.; Mines, A.; Mishra, S.; Clark, L.; Hofman, P.; Rashidian, M. Nanobodies for Medical Imaging: About Ready for Prime Time? Biomolecules, 11, 637 (2021). https://doi.org/10.3390/biom11050637.
11. Chen, X., Zaro, J. and Shen W-C. Fusion Protein Linkers: Property, Design and Functionality. Adv Drug Deliv Rev. 65(10): 1357-1369 (2013).

## Claims

1. **A truncated progranulin protein or a variant thereof,** wherein said truncated progranulin protein is N-terminally truncated compared to amino acid sequence according to SEQ ID NO. 1.

2. The truncated progranulin protein of claim 1, wherein said truncated progranulin protein has 400 - 500 amino acids truncated at its N-terminus as compared with wild type progranulin (SEQ ID NO. 1) and/or
wherein said truncated progranulin comprises an amino acid sequence which is at least 80%, 85%, 90%, 95%, 98% or 99% identical to SEQ ID NO: 2 or 3, or comprises the amino acid sequence of SEQ ID NO: 2 or 3, or a functional fragment thereof and/or
wherein the protein is not a full length progranulin protein.

3. The truncated progranulin protein or variant thereof, of claim 1 or 2, comprising at least granulin domains D and/or E; and preferably does not comprise one of domains G, F, B, A, C, more preferably does not comprise the domains G, F, B, A, and C (as shown in Figure 1).

4. **A fusion protein** comprising a truncated progranulin protein (or a progranulin fragment) fused to a non-progranulin protein, wherein the truncated progranulin protein or progranulin fragment is the truncated progranulin protein or a variant thereof recited in any one of claims 1 to 3.

5. The fusion protein of claim 4, wherein the non-progranulin protein is fused to the truncated progranulin protein by a linker, such as a GS linker; preferably wherein (a) the N-terminal end of the truncated progranulin protein is fused to the C-terminus of the non-progranulin protein or (b) the C-terminal end of the truncated progranulin protein is fused to the N-terminus of the non-progranulin protein.

6. The fusion protein of claim 4 or 5, wherein the non-progranulin protein is selected from an antigen binding protein, an enzyme, a structural protein, a membrane anchor, a ligand binding domain or a receptor binding domain, preferably the non-progranulin protein is an antigen binding region wherein the antigen binding region is selected from the group consisting of an antibody, a minibody, a diabody, an antigen-binding fragment (Fab), a single-chain variable fragment (scFv), a heavy-chain only antibody, a single domain antibody (nanobody), a chimeric antibody, or any antigen binding fragments thereof and/or wherein the antigen-binding region is obtained from an antigen-binding region library.

7. **A fusion protein** comprising
- an antigen binding region,
- optionally linked by a linker region with
- a progranulin domain comprising the sequence of the truncated progranulin protein or a variant thereof of claims 1 to 3,
wherein the progranulin domain specifically binds to sortilin.

8. The fusion protein of any one of claims 4 to 7, wherein the antigen binding region comprises
(a) a human FcRn-binding domain which has a human FcRn-binding activity at pH 5.8 to pH 6.0 and at pH 7.4, wherein the human FcRn-binding activity at pH 7.0 and at 25°C is stronger than KD 3.2 mM, and wherein the human FcRn-binding domain is an Fc domain resulting from substituting a different amino acid for at least one amino acid in the Fc domain of a human IgG1, IgG2, IgG3 or IgG4; and
(b) an antigen-binding domain which has a lower antigen-binding activity at pH 5.8 to pH 6.0 than at pH 7.4 and wherein the ratio of antigen-binding activity at pH 5.8 and at pH 7.4 is 2 or greater, 10 or greater or 40 or greater in the value of KD (at pH 5.8)/KD (at pH 7.4).

9. **A nucleic acid** sequence encoding the truncated progranulin protein of any one of claims 1 to 3 or the fusion protein of any one of claims 4 to 8.

10. **A vector** comprising the nucleic acid sequence of claim 9, preferably an expression vector, more preferably comprising a promoter sequence operably linked to the nucleic acid sequence of claim 10.

11. **A host cell** comprising the truncated progranulin protein of any one of claims 1 to 3, the fusion protein of any one of claims 4 to 8, the nucleic acid sequence of claim 9, or the vector of claim 10, preferably a host cell having the nucleic acid sequence of claim 9 integrated in its genome.

12. **A pharmaceutical composition comprising:**
(i) the truncated progranulin protein of any one of claims 1 to 3, the fusion protein of any one of claims 4 to 8, the nucleic acid sequence of claim 9, or the vector of claim 10, or the host cell of claim 11, and
(ii) a pharmaceutically acceptable carrier, stabilizer and/or excipient.

13. **A compound for use in the treatment of** a **disease,** the compound being selected from the truncated progranulin protein of any one of claims 1 to 3, the fusion protein of any one of claims 4 to 8, the nucleic acid sequence of claim 9, or the vector of claim 10, or the host cell of claim 11, or the pharmaceutical composition of claim 12.

14. **A use of the fusion protein** of any one of claims 4 to 8 for increasing protein degradation of a target protein, preferably wherein the fusion protein comprises at least one antigen binding domain specifically binding to the target protein.

15. **A method for screening a candidate fusion protein for an improved effect on lysosome-dependent protein degradation of a target protein,** the method comprising:
(a) providing a candidate fusion protein comprising the fusion protein of any one of claims 4 to 8 with at least one amino acid mutation in the progranulin domain,
(b) contacting the candidate fusion protein and a target protein under conditions enabling degradation of the target protein by lysosomes,
(c) determining the amount or level of target protein degradation over time compared to a control or reference,
wherein an increased target protein degradation compared to the control or reference indicates that the at least one amino acid mutation in the progranulin domain in the candidate fusion protein has an effect on lysosome-dependent protein degradation of a target protein.
